# EUROPÄISCHE PATENTSCHRIFT

(11) **EP 0 368 342 B1**
(45) Veröffentlichungstag und Bekanntmachung des Hinweises auf die Patenterteilung: **14.09.1994**
(21) Anmeldenummer: 89120894.4
(22) Anmeldetag: 10.11.1989
(51) Int. Cl.: C12N 15/70, C12N 15/58, C12N 15/49

(54) **Herstellung und Verwendung einer DNA zur Erhöhung der Ausbeute bei der Expression von rekombinanten Genen**
Process for producing and use of a DNA to increase the productivity in the expression of recombinant genes
Procédé de préparation et utilisation d'un ADN et son application pour augmenter la productivité d'expression de gènes recombinants

(30) Priorität: 11.11.1988 DE 3838377
(43) Veröffentlichungstag der Anmeldung: 16.05.1990
(73) Patentinhaber: BOEHRINGER MANNHEIM GMBH, 68298 Mannheim (DE)
(72) Erfinder: Brinkmann, Ulrich, D-4700 Hamm (DE); Mattes, Ralf, D-7000 Stuttgart 75 (DE); Buckel, Peter, D-8131 Bernried (DE)
(74) Vertreter: Huber, Bernhard, Dipl.-Chem.

(56) Entgegenhaltungen:
- EP-A- 0 373 365
- GB-A- 2 135 677
- GB-A- 2 172 001
- CELL, Band 45, 9. Mai 1986, Seiten 453-459, Cambridge, US; G.M. GARCIA et al.:"The E. coli dnaY gene encodes an arginine transfer RNA"

## Beschreibung

Die Erfindung betrifft die Verwendung einer expressionsverstärkenden DNA zur Erhöhung der Ausbeute bei der Expression eines rekombinanten Gens durch Transformation von geeigneten Wirtszellen mit einem das rekombinante Gen enthaltenden Expressionsvektor.

Garcia et al. (Cell Vol. 45 (1986), 453-459) offenbaren, daß es sich bei dem E.coli dnaY-Genprodukt um eine Arginin-tRNA handelt. Seine 77 Nukleotide lange Sequenz kann zu einer typischen Kleeblattstruktur mit einem UCU-Antikodon gefaltet werden, welches dem seltenen Arginin-Kodon AGA entspricht. Ein dnaY⁺-Plasmid führt zur Überproduktion von mindestens einer Arginin-akzeptierenden Spezies von tRNA^{Arg}.

GB-A-21 35 677 betrifft die extrachromosomale Expression von tRNAs, insbesondere von tRNA^{Trp}, und deren Wirkung auf die Supplementation von Nonsense-Kodon-Mutationen.

Bei der Expression rekombinanter Gene, und vor allem eukaryontischer Gene in E. coli stellt sich oft das Problem einer mangelnden Expression des gewünschten Genproduktes. Dies wird von den auf diesem Gebiet tätigen Forschern auf verschiedene Ursachen zurückgeführt, unter anderem auf eine nur geringe Fermentationsrate von mit bestimmten rekombinanten Genen transformierten E. coli-Zellen. Zwar weisen diese Zellen teilweise eine recht gute Expression des rekombinanten Gens auf, wachsen jedoch schlecht, wodurch nur eine geringe Biomasse erhalten wird und dadurch auch eine nur geringe Ausbeute an dem gewünschten Genprodukt. Der Erfindung lag daher die Aufgabe zugrunde, ein Verfahren und Mittel bereitzustellen, die Ausbeute an einem gewünschten Genprodukt bei der Expression in E. coli zu erhöhen.

Diese Aufgabe wird erfindungsgemäß gelöst durch die Verwendung einer expressionsverstärkenden DNA, die eine nach Transkription zur Ausbildung einer t-RNA-Kleeblattstruktur befähigte DNA-Sequenz aufweist und in dem Bereich der DNA, der nach Transkription in der Kleeblattstruktur die Antikodonschleife bildet, mit einem Oligonukleotid der Sequenz 5'-GACTTAGAAGGTCGTT-3' oder der dazu komplementären Sequenz 5'-AACGACCTTCTAAGTC-3' hybridisiert, zur Erhöhung der Ausbeute bei der Expression eines rekombinanten Gens durch Transformation von E.coli-Wirtszellen mit einem das rekombinante Gen enthaltenden Expressionsvektor, dadurch gekennzeichnet, daß man die expressionsverstärkende DNA in exprimierbarer Form ebenfalls in die Wirtszellen einbringt und exprimiert.

Die Kleeblattstruktur von t-RNA-Molekülen wird durch Basenpaarungen in bestimmten Bereichen des einzelsträngigen RNA-Moleküls bewirkt, wobei bei allen t-RNA-Molekülen bestimmte Regelmäßigkeiten auftreten. In jeder dieser Kleeblattstrukturen findet man eine linke Schleife, die sogenannte Dihydrouridinschleife, eine zweite Schleife, die sogenannte Antikodonschleife, welche bei t-RNA-Molekülen mit einem komplementären Triplett auf der mRNA Basenpaarungen eingehen kann und dadurch die Spezifität der t-RNA bestimmt, sowie eine rechts gelegene Schleife, die sogenannte Pseudouridinschleife und zwischen der Antikodonschleife und der Pseudouridinschleife häufig eine Extraschleife, die variabel ist. Die expressionsverstärkende DNA hat eine DNA-Struktur, deren RNA nach der Transkription eine derartige Kleeblattstruktur bilden kann. Es sind daher erfindungsgemäß natürliche und synthetische Gene eingeschlossen, die zur Bildung der Kleeblattstruktur fähig sind, wobei auch DNA-Sequenzen im Rahmen der Erfindung liegen, welche über die zur Ausbildung der Kleeblattstruktur befähigte DNA-Sequenz hinaus weitere Sequenzen enthalten und unter Bildung einer RNA mit Kleeblattstruktur prozessiert werden können. Charakterisierend für die expressionsverstärkende DNA ist jedoch, daß sie an der Antikodonschleife mit dem genannten Oligonukleotid hybridisiert. In einer bevorzugten Ausführungsform der Erfindung hybridisiert eine expressionsverstärkende DNA mit einem Oligonukleotid der Sequenz 5'-CACGACTTAGAAGGTCGTTG-3' oder der dazu komplementären Sequenz (5'-CAACGACCTTCTAAGTCGTG-3').

Eine derartige expressionsverstärkende DNA kann sowohl synthetisch hergestellt werden, als auch z.B. aus E. coli- Zellen isoliert werden. In einer bevorzugten Ausführungsform der Erfindung wird zur Herstellung einer expressionsverstärkenden DNA eine Genbank aus mit PstI partiell verdauter chromosomaler E. coli-DNA in einem geeigneten Vektor angelegt, E. coli-Zellen mit den Genbankvektoren und einem Expressionsplasmid, das ein rekombinantes Gen enthält, bei dessen Expression die E. coli-Zellen schlecht wachsen (wobei der Ausdruck "schlecht wachsen" im Rahmen der Erfindung bedeuten soll, daß die Zellen bei Expression des rekombinanten Gens alleine schlechter wachsen als bei zusätzlicher Expression der expressionsverstärkenden DNA, cotransfiziert, selektiert und induziert, kultiviert, Klone isoliert, die zu großen Kolonien angewachsen sind, aus diesen Klonen die Genbank-Vektoren isoliert. Durch Hybridisierung mit einem Oligonukleotid der Sequenz 5'-GACTTAGAAGGTCGTT-3' der dazu komplementären Sequenz (5'-AACGACCTTCTAAGTC-3') kann die Anwesenheit der expressionsverstärkenden DNA überprüft werden. In einer besonders bevorzugten Ausführungsform der Erfindung verwendet man ein Oligonukleotid der Sequenz 5'-CACGACTTAGAAGGTCGTTG-3' oder dem dazu komplementären Oligonukleotid (5'-CAACGACCTTCTAAGTCGTG-3').

Das erfindungsgemäß bevorzugte Herstellungsverfahren kann mit Expressionsplasmiden durchgeführt werden, die ein rekombinantes Gen enthalten, bei dessen Expression in E. coli die Zellen nur schlecht wachsen. Hierzu wird vorzugsweise ein induzierbares Expressionsplasmid verwendet, so daß bis zur Induktion eine normale Fermentation der E. coli-Zellen stattfinden kann. Nach der Induktion wachsen solche Klone, die einen Genbankvektor enthalten, der wiederum eine expressionsverstärkende DNA enthält, zu großen Kolonien an. Dies kann durch Hybridisierung mit einem Oligonukleotid der obengenannten Sequenz überprüft werden. Aus den Klonen mit dem Genbankvektor, der die expressionsverstärkende DNA enthält, kann diese sodann durch Verdauung mit den zur Herstellung der Genbank verwendeten Restriktionsenzymen isoliert werden.

In einer bevorzugten Ausführungsform der Erfindung verwendet man als Expressionsplasmid ein t-PA-Expressionsplasmid, und besonders bevorzugt das Plasmid pUBS98.sl (siehe Beispiel 3).

Das erfindungsgemäß verwendete induzierbare Expressionsplasmid wird vorzugsweise durch Isopropyl-β-D-thiogalactopyranosid, besonders bevorzugt in einer Menge von 5 bis 20 mmol/l induziert.

Zur Herstellung der Genbank verwendet man erfindungsgemäß vorzugsweise den Vektor pACYC177, DSM 3693P.

Ein weiterer Gegenstand der Erfindung ist die Verwendung einer expressionsverstärkenden DNA zur Erhöhung der Ausbeute bei der Expression eines rekombinanten Gens durch Transformation von E. coli-Zellen mit einem das rekombinante Gen enthaltenden Expressionsvektor, wobei man die expressionsverstärkende DNA in exprimierbarer Form ebenfalls in die Wirtszellen einbringt und ihre Expression vor oder gleichzeitig mit der des Expressionsvektors bewirkt.

In einer bevorzugten Ausführungsform der Erfindung bringt man die expressionsverstärkende DNA in den das rekombinante Gen enthaltenden Expressionsvektor ein. Dies geschieht erfindungsgemäß derart, daß die expressionsverstärkende DNA entweder unter der Kontrolle eines eigenen separaten Promotors steht, oder zusammen mit dem rekombinanten Gen unter Kontrolle desselben Promotors steht.

In einer anderen bevorzugten Ausfügrungsform der Erfindung bringt man die expressionsverstärkende DNA auf einem zu dem das rekombinante Gen enthaltenden Expressionsvektor kompatiblen Vektor ein. Unter einem kompatiblen Vektor wird im Sinne der Erfindung ein Vektor verstanden, der einen von dem Expressionsvektor verschiedenen Replikationsursprung besitzt. Hierdurch wird eine gleichzeitige Replikation und Transkription beider Plasmide in einer Zelle ermöglicht.

Vorzugsweise verwendet man als die expressionsverstärkende DNA enthaltenden Vektor das Plasmid pUBS 100 und als das rekombinante Gen enthaltenden Expressionsvektor einen dazu kompatiblen Vektor, z.B. das t-PA exprimierende Plasmid pUBS 98.s1. In einer bevorzugten Ausführungsform der Erfindung exprimiert man als rekombinantes Gen das Gen bzw. die cDNA für t-PA oder ein t-PA-Derivat, Urokinase oder ein HIV-Protein.

Die Expression der expressionsverstärkenden DNA kann erfindungsgemäß vor oder gleichzeitig mit dem das rekombinante Gen enthaltenden Expressionsvektor stattfinden, wobei jedoch die Expression nicht so lange vor der des Expressionsvektors stattfinden darf, daß das Genprodukt der expressionsverstärkenden DNA durch Nukleasen in der Wirtszelle in wesentlichem Umfang abgebaut wird. Es ist daher erfindungsgemäß bevorzugt, die Expression gleichzeitig mit der des Expressionsvektors besonders bevorzugt durch konstitutive Expression oder Induktion zu bewirken. Hierzu kann die expressionsverstärkende DNA erfindungsgemäß z.B. unter der Kontrolle eines induzierbaren Promotors, vorzugsweise des lac-Promotors stehen.

Die folgenden Beispiele erläutern die Erfindung weiter.

### Beispiel 1

Klonierung und Selektion einer expressionsverstärkenden DNA: Mit allgemein bekannten molekularbiologischen Mitteln zusammenfassend s. u.a. Winnacker E.L., Gene und Klone, VCH-Verlag 1985) wurde eine Genbank von partiell mit dem Restriktionsenzym PstI verdauter E. coli-DNA in pACYC177, DSM 3693P, (Chang und Cohen, J. Bacteriol. 134 (1978), 1141-1156) angelegt. Dazu wurde PstI-geschnittene pACYC177-Plasmid-DNA mit partiell PstI-verdauter chromosomaler E. coli-DNA ligiert. Klone, welche ein Insert in der PstI-Site von pACYC-177 enthalten, können durch Ampicillinsensibilität identifiziert werden, sowie durch ein um die Länge des Insertes vergrößertes Molekulargewicht. Die Plasmid-DNA derartiger Klone kann durch Gelelektrophorese/Gelelution von Plasmid-DNA ohne Insert abgetrennt und isoliert werden. Aus dieser Genbank wurde durch Cotransformation der Genbankplasmide mit dem t-PA-Expressionsplasmid pUBS98.sl (Herstellung siehe Beispiel 3) und Selektion auf LB-Kanamycin/Ampicillin-Platten, welche 10 mmol/l IPTG (Isopropyl-β-D-thiogalactopyranosid) enthielten, das Plasmid pUBS100 isoliert. Dieses Plasmid pUBS100 enthält ein ca. 3000 Basen langes Stück chromosomaler E. coli-DNA, welches bewirkt, daß die E. coli-Zellen, DSM 3689, die das Plasmid pUBS100 und das Expressionsplasmid pUBS 98.s1 tragen und durch IPTG-Induktion t-PA produzieren, im Gegensatz zu E. coli-Zellen mit pUBS 98.s1 und pACYC 177 nach der Induktion zu großen Kolonien anwachsen können.

### Beispiel 2

Expression von t-PA in E. coli durch Cotransformation mit einem die expressionsverstärkende DNA enthaltenden Plasmid.

In diesem Beispiel wurde als das die expressionsverstärkende DNA enthaltende Plasmid das Plasmid pUBS100 verwendet. Die Expression bei Cotransformation von E. coli, DSM 3689, mit pUBS100 und dem t-PA-Expressionsplasmid pUBS98.s1, wurde mit der Expressionsrate in denselben Wirtszellen bei Transformation mit dem Plasmid pUBS98.s1 alleine verglichen. Weiter wurde die Expressionsrate mit der des Plasmids pePa98.1 (DE 36 13 401) in demselben Stamm verglichen. Die Ergebnisse dieser Vergleiche sind in Tabelle 1 dargestellt. Weiter wurde die Expression von t-PA unter Verwendung eines Plasmides, das die expressionsverstärkende DNA und das t-PA-Gen enthält, nämlich dem Plasmid pUBS98.sky, DSM 4898, untersucht. Auch hierfür ist das Ergebnis in der Tabelle 1 dargestellt.

**Tabelle 1**

| E. coli, DSM 3689 | % t-PA intakt/Gesamtprotein | Vitalität |
|---|---|---|
| + pePa 98.1 | 3% | eingeschränkt |
| + pUBS 98.s1 | 10% | sehr schlecht |
| + pUBS 98.s1 + pUBS 100 | 30% | sehr gut |
| + pUBS 98.sky | 30% | sehr gut |

Aus den in Tabelle 1 gezeigten Ergebnissen kann deutlich ersehen werden, daß die Expressionsrate von t-PA, welches in E. coli-Zellen exprimiert wird, die zusätzlich die expressionsverstärkende DNA enthalten, gegenüber Zellen, die die expressionsverstärkende DNA nicht enthalten, deutlich erhöht ist. Dies korreliert mit der Vitalität der erhaltenen Klone.

### Beispiel 3

Als Ausgangsplasmid zur Konstruktion des Plasmids pUBS98.s1 wurde das Plasmid pePa98.1 (EP-A-242 836) verwendet. Die ca. 400 Basenpaare lange 3'-untranslantierte Region der t-PA-cDNA in diesem Plasmid wurde durch Deletion eines 361 Basenpaare langen XhoII-Fragments auf ca. 40 Basenpaare verkürzt. Das resultierende Plasmid erhielt den Namen pePa126.1 und kann von pePa98.1 z.B. dadurch unterschieden werden, daß bei einem Doppelverdau dieser Plasmide mit den Restriktionsendonukleasen BamHI und HindIII bei pePa98.1 zwei Fragmente der Länge 2234 Basenpaare und 4372 Basenpaare nachgewiesen werden können, bei Plasmid pePa126.1 hingegen zwei Fragmente der Länge 1873 Basenpaare und 4372 Basenpaare. Dieses Plasmid pePa126.1 wurde an der singulären HindIII-Schnittstelle linearisiert und die überstehenden Enden mit Klenow-Enzym und dNTP's vollständig aufgefüllt. Weiter wurde ebenfalls aus dem Plasmid pePa126.1 ein 472 Basenpaare langes EcoR1-Fragment isoliert und mit S1-Nuklease behandelt. Durch Ligation der beiden Fragmente aus dem Plasmid pePa126.1 wurde das Plasmid pUBS98.s1 erhalten. pUBS 98.s1 kann von pePa126.1 durch Restriktionsanalyse unterschieden werden: BanII verdaute pePa126.1 DNA ist definiert durch Fragmente der Länge 1175 Basenpaare, 393 Basenpaare, 14 Basenpaare, 165 Basenpaare und 4560 Basenpaare; pUBS98.s1-DNA ist dadurch charakterisiert, daß BanII-Fragmente die Länge von ca. 1175 Basenpaare, 393 Basenpaare, 14 Basenpaare, 165 Basenpaare, 470 Basenpaare und 4540 Basenpaare aufweisen.

## Patentansprüche

1. Verwendung einer expressionsverstärkenden DNA, die eine nach Transkription zur Ausbildung einer t-RNA-Kleeblattstruktur befähigte DNA-Sequenz aufweist und in dem Bereich der DNA, der nach Transkription in der Kleeblattstruktur die Antikodonschleife bildet, mit einem Oligonukleotid der Sequenz 5'-GACTTAGAAGGTCGTT-3' oder der dazu komplementären Sequenz 5'-AACGACCTTCTAAGTC-3' hybridisiert, zur Erhöhung der Ausbeute bei der Expression eines rekombinanten Gens durch Transformation von E.coli-Wirtszellen mit einem das rekombinante Gen enthaltenden Expressionsvektor,
**dadurch gekennzeichnet,**
daß man die expressionsverstärkende DNA in exprimierbarer Form ebenfalls in die Wirtszellen einbringt und exprimiert.

2. Verwendung nach Anspruch 1,
**dadurch gekennzeichnet,**
daß die expressionsverstärkende DNA mit dem Oligonukleotid der Sequenz 5'-CACGACTTAGAAGGTCGTTG-3' oder dem dazu komplementären Oligonukleotid (5'-CAACGACCTTCTAAGTCGTG-3') hybridisiert.

3. Verwendung nach Anspruch 1 oder 2,
**dadurch gekennzeichnet,**
daß man als rekombinantes Gen das Gen für t-PA oder ein t-PA-Derivat, Urokinase oder ein HIV-Protein exprimiert.

4. Verfahren zur Herstellung einer expressionsverstärkenden DNA, die eine nach Transkription zur Ausbildung einer t-RNA-Kleeblattstruktur befähigte DNA-Sequenz aufweist und in dem Bereich der DNA, der nach Transkription in der Kleeblattstruktur die Antikodonschleife bildet, mit einem Oligonukleotid der Sequenz 5'-GACTTAGAAGGTCGTT-3' oder der dazu komplementären Sequenz (5'-AACGACCTTCTAAGTC-3') hybridisiert,
**dadurch gekennzeichnet,**
daß man eine Genbank aus mit PstI partiell verdauter chromosomaler E.coli DNA in einem geeigneten Vektor anlegt, E.coli-Zellen mit den Genbankvektoren und einem Expressionsplasmid, das ein rekombinantes Gen enthält, bei dessen Expression die E.coli-Zellen schlecht wachsen, cotransfiziert, unter Selektionsbedingungen kultiviert, Klone isoliert, die zu großen Kolonien angewachsen sind, aus diesen Klonen die Genbankvektoren isoliert und durch Hybridisierung mit einem Oligonukleotid der Sequenz 5'-GACTTAGAAGGTCGTT-3' oder der dazu komplementären Sequenz (5'-AACGACCTTCTAAGTC-3') auf Anwesenheit der expressionsverstärkenden DNA untersucht und diese isoliert.

5. Verfahren nach Anspruch 4,
**dadurch gekennzeichnet,**
daß man ein Oligonukleotid der Sequenz 5'-CACGACTTAGAAGGTCGTTG-3' oder das dazu komplementäre Oligonukleotid (5'-CAACGACCTTCTAAGTCGTG-3') verwendet.

6. Verfahren nach Anspruch 4 oder 5,
**dadurch gekennzeichnet,**
daß man ein induzierbares Expressionsplasmid verwendet.

7. Verfahren nach Anspruch 4, 5 oder 6,
**dadurch gekennzeichnet,**
daß man ein t-PA-Expressionsplasmid verwendet.

8. Verfahren nach Anspruch 6 oder 7,
**dadurch gekennzeichnet,**
daß man mit Isopropyl-β-D-thiogalactopyranosid, vorzugsweise in einer Menge von 5 bis 20 mmol/l induziert.

9. Verfahren nach einem der Ansprüche 4 bis 8,
**dadurch gekennzeichnet,**
daß man als Vektor zur Anlage der Genbank pACYC177 (DSM 3693P) oder ein verwandtes Derivat verwendet.

## Claims

1. Use of an expression-enhancing DNA which has a DNA sequence capable of forming a t-RNA clover-leaf structure after transcription and hybridizes in that region of the DNA which after transcription, forms the anticodon loop in the clover-leaf structure with an oligonucleotide with the sequence 5'-GACTTAGAAGGTCGTT-3' or its complementary sequence (5'-AACGACCTTCTAAGTC-3') in order to increase the yield when expressing a recombinant gene by transformation of E. coli host cells with an expression vector containing the recombinant gene,
**wherein**
the expression-enhancing DNA is like-wise introduced into the host cells in an expressionable form and expressed.

2. Use as claimed in claim 1,
**wherein**
the expression-enhancing DNA it hybridized with the oligonucleotide having the sequence 5'-CACGACTTAGAAGGTCGTTG-3' or its complementary oligonucleotide (5'-CAACGACCTTGTAAGTCGTG-3').

3. Use as claimed in claim 1 or 2,
**wherein**
the gene for t-PA or a t-PA derivative, urokinase or a HIV protein is expressed as the recombinant gene.

4. Process for the production of an expressionenhancing DNA which has a DNA sequence capable of forming a t-RNA clover-leaf structure after transcription and hybridizes in that region of the DNA which, after transcription, forms the anticodon loop in the clover-leaf structure with an oligonucleotide with the sequence 5'-GACTTAGAAGGTCGTT-3' or its complementary sequence (5'-AACGACCTTCTAAGTC-3') in order to increase the yield when expressing a recombinant gene by transformation of E. coli host cells with an expression vector containing the recombinant gene,
**wherein**
a gene bank of chromosomal E. coli DNA partially digested with PstI is set up in a suitable vector, E. coli cells are co-transfected with the gene bank vectors and an expression plasmid which contains a recombinant gene on the expression of which the E. coli cells grow poorly, the cells are cultivated under selection conditions, clones are isolated which have grown to large colonies and the gene bank vectors are isolated from these clones and assayed for the presence of the expression-enhancing DNA by hybridization with an oligonucleotide with the sequence 5'-GACTTAGAAGGTCGTT-3' or its complementary sequence (5'-AACGACCTTCTAAGTC-3') and this is isolated.

5. Process as claimed in claim 4,
**wherein**
an oligonucleotide with the sequence 5'-CACGACTTAGAAGGTCGTTG-3' or its complementary oligonucleotide (5'-CAACGACCTTGTAAGTCGTG-3') is used.

6. Process as claimed in claim 4 or 5,
**wherein**
an inducible expression plasmid is used.

7. Process as claimed in claim 4, 5 or 6,
**wherein**
a t-PA expression plasmid is used.

8. Process as claimed in claim 6 or 7,
**wherein**
one induces with isopropyl-β-D-thiogalactopyranoside preferably in an amount from 5 to 20 mmol/l.

9. Process as claimed in one of the claims 4 to 8,
**wherein**
pACYC177 (DSM 3693P) or a related derivative is used as the vector to set up the gene bank.

## Revendications

1. Utilisation d'un ADN renforçant l'expression qui comporte une séquence d'ADN capable de former une structure d'ARNt en feuille de trèfle après la transcription et qui, dans le domaine de l'ADN qui forme la boucle de l'anticodon dans la structure en feuille de trèfle après la transcription, s'hybride avec un oligonucléotide de séquence 5'-GACTTAGAAGGTCGTT-3' ou de séquence 5'-AACGACCTTCTAAGTC-3' complémentaire de la précédente, pour augmenter le rendement lors de l'expression d'un gène recombinant par transformation de cellules hôtes de E. coli avec un vecteur d'expression contenant le gène recombinant, caractérisée en ce que l'on introduit également l'ADN renforçant l'expression sous forme exprimable dans les cellules hôtes et on l'exprime.

2. Utilisation selon la revendication 1, caractérisée en ce que l'ADN renforçant l'expression s'hybride avec l'oligonucléotide de séquence 5'-CACGACTTAGAAGGTCGTTE-3' ou avec l'oligonucléotide (5'-CAACGACCTTCTAAGTCGTG-3') complémentaire du précédent.

3. Utilisation selon la revendication 1 ou 2, caractérisée en ce que l'on exprime comme gène recombinant le gène du t-PA ou d'un dérivé du t-PA, de l'urokinase ou d'une protéine du VIH.

4. Procédé de préparation d'un ADN renforçant l'expression qui comporte une séquence d'ADN capable de former une structure d'ARNt en feuille de trèfle après la transcription et qui s'hybride, dans le domaine de l'ADN qui forme la boucle de l'anticodon dans la structure en feuille de trèfle après la transcription, avec un oligonucléotide de séquence 5'-GACTTAGAAGGTCGTT-3' ou de séquence (5'-AACGACCTTCTAAGTC-3') complémentaire de la précédente, caractérisé en ce que l'on intègre une banque de gènes constituée par de l'ADN chromosomique de E. coli partiellement digéré avec PstI dans un vecteur approprié, on cotransfecte des cellules de E. coli avec les vecteurs de la banque de gènes et un plasmide d'expression qui contient un gène recombinant lors de l'expression duquel les cellules de E. coli poussent mal, on cultive dans des conditions de sélection, on isole les clones qui se sont développés en colonies de grande taille, on isole les vecteurs de la banque de gènes à partir de ces clones et on les examine par hybridation avec un oligonucléotide de séquence 5'-GACTTAGAAGGTCGTT-3' ou de séquence (5'-AACGACCTTCTAAGTC-3') complémentaire de la précédente en ce qui concerne la présence de l'ADN renforçant l'expression et on isole celui-ci.

5. Procédé selon la revendication 4, caractérisé en ce que l'on utilise un oligonucléotide de séquence 5'-CACGACTTAGAAGGTCGTTG-3' ou l'oligonucléotide (5'-CAACGACCTTCTAAGTCGTG-3') complémentaire du précédent.

6. Procédé selon la revendication 4 ou 5, caractérisé en ce que l'on utilise un plasmide d'expression inductible.

7. Procédé selon la revendication 4, 5 ou 6, caractérisé en ce que l'on utilise un plasmide d'expression du t-PA.

8. Procédé selon la revendication 6 ou 7, caractérisé en ce que l'on induit avec l'isopropyl-β-D-thiogalactopyranoside, de préférence en une quantité de 5 à 20 mmol/l.

9. Procédé selon l'une des revendications 4 à 8, caractérisé en ce que l'on utilise pACYC177 (DSM 3693P) ou un dérivé apparenté comme vecteur pour l'intégration de la banque de gènes.
